# EUROPÄISCHE PATENTSCHRIFT

(11) **EP 3 661 449 B1**
(45) Veröffentlichungstag und Bekanntmachung des Hinweises auf die Patenterteilung: **09.10.2024**
(21) Anmeldenummer: 18762009.1
(22) Anmeldetag: 31.07.2018
(51) Int. Cl.: A61B 50/30, A61L 2/02

(54) **MODULAR AUFGEBAUTER DECKEL FÜR EINEN STERILBEHÄLTER UND FILTERABDECKUNG FÜR EINEN SOLCHEN DECKEL**
MODULARLY STRUCTURED LID FOR A STERILE CONTAINER AND FILTER COVERING FOR SUCH A LID
COUVERCLE DE CONCEPTION MODULAIRE POUR CONTENEUR DE STÉRILISATION ET CACHE FILTRANT D'UN TEL COUVERCLE

(30) Priorität: 03.08.2017 DE 102017117624
(43) Veröffentlichungstag der Anmeldung: 10.06.2020
(73) Patentinhaber: Aesculap AG, 78532 Tuttlingen (DE)
(72) Erfinder: HENKE, Matthias, 78567 Fridingen (DE); ELISCH, Andreas, 78655 Dunningen (DE); SCHWEIZER, Matthias, 78532 Tuttlingen (DE); THOMAS, Stefan, 78532 Tuttlingen (DE); GRAY-DREIZLER, John, 78078 Niedereschach (DE); BOHNENSTENGEL, Philipp, 78256 Steißlingen (DE); STERK, Thomas, 78224 Singen (DE); BERNAUER, Betina, 78187 Geisingen (DE)
(74) Vertreter: Winter, Brandl - Partnerschaft mbB
(86) Internationale Anmeldenummer: PCT/EP2018/070732
(87) Internationale Veröffentlichungsnummer: WO 2019/025441

(56) Entgegenhaltungen:
- EP-A1- 2 179 746
- WO-A1-03/041604
- DE-A1- 102004 020 803
- DE-B3- 102004 020 805

## Beschreibung

Die Erfindung betrifft einen modular aufgebauten Deckel für einen Sterilbehälter, einen Sterilbehälter mit einem solchen Deckel, sowie eine Filterabdeckung für einen solchen Deckel.

### Hintergrund der Erfindung

Sterilbehälter sind grundsätzlich aus dem Stand der Technik bekannt und werden in der Medizin insbesondere genutzt, um chirurgische Instrumente, Implantate und dergleichen zu sterilisieren und nach der Sterilisation kurzzeitig zu lagern bzw. zu transportieren. Zu sterilisierende Gegenstände werden dabei generell zunächst in den Sterilbehälter bzw. in ein wannenartiges erstes Behälterteil des Sterilbehälters eingelegt. Anschließend wird ein deckelartiges zweites Behälterteil bzw. ein Deckel an bzw. auf dem wannenartigen ersten Behälterteil angeordnet und die beiden Behälterteile werden zueinander verschlossen. Der verschlossene Sterilbehälter wird einem Sterilisator zugeführt. In diesem werden die in dem Behälterinnenraum befindlichen zu sterilisierenden Gegenstände in einem beliebigen Sterilisationsverfahren (z.B. Heißluftsterilisation, Wasserdampfsterilisation, etc.) sterilisiert.

### Stand der Technik

Aus dem Stand der Technik ist es bekannt, Sterilbehälter mit Filtereinrichtungen/Filter auszurüsten, welche ein Eindringen von Keimen, Bakterien oder Ähnlichem in den Sterilbehälter verhindern und einen sterilen Medienaustausch während einer Sterilisation ermöglichen sollen. Die Filtereinrichtungen sind dabei häufig von einer Sterilbehälterinnenseite/ von innen montierbar und demontierbar und an einer Innenseite des Deckels an einem, insbesondere perforierten, Gasaustauschabschnitt des Deckels angeordnet. An einer Außenseite des Deckels ist der Gasaustauschabschnitt von einer Außenabdeckung/Filterabdeckung abgedeckt, welche einem Schutz der Filtereinrichtung vor mechanischen Einflüssen, beispielsweise während eines Transports oder einer Lagerung, dient.

In den aus dem Stand der Technik bekannten Sterilbehälterdeckeln werden die Filterabdeckungen grundsätzlich werkseitig bereits an Deckelbauteilen des Sterilbehälterdeckels montiert bzw. befestigt. Diese bekannten integralen Lösungen für einen Sterilbehälterdeckel sind für einen Anwender häufig schwierig handhabbar, insbesondere da die Filterabdeckung nicht einfach von dem Deckelbauteil abgenommen werden kann und der Filter für einen Anwender von außen nicht überprüft! eingesehen werden kann. Von innen montierte Filtereinrichtungen werden daher häufig erst nach einer Entnahme der sterilisierten Gegenstände durch einen sterilen Anwender auf mögliche Beschädigungen überprüft. Wird dann festgestellt, dass die Filtereinrichtung beschädigt oder überhaupt nicht vorhanden ist, müssen die entnommenen Gegenstände erneut sterilisiert werden, ein möglicherweise kontaminierter Instrumententisch, auf welchem die entnommenen Gegenstände in der Zwischenzeit abgelegt wurden, muss gereinigt werden und der sterile Anwender muss sich gegebenenfalls umziehen.

Aus der EP 2 179 746 A1 ist ein Deckel gemäß dem Oberbegriff des Anspruchs 1, sowie eine Filterabdeckung gemäß dem Oberbegriff des Anspruchs 8 bekannt. Weiterer relevanter Stand der Technik findet sich in der WO 03/041604 A1, der DE 10 2004 020 803 A1 und der DE 10 2004 020 805 B3.

### Kurzbeschreibung der Erfindung

Vor diesem Hintergrund ist es Aufgabe der vorliegenden Erfindung, die genannten Nachteile aus dem Stand der Technik zu vermeiden bzw. wenigstens zu mildern. Insbesondere soll ein Sterilbehälter bzw. ein Sterilbehälterdeckel bereitgestellt werden, welcher für einen Anwender in einfacherer Weise handhabbar ist und welcher ein Überprüfen einer Filtereinrichtung/ eines Filters ohne vorheriges Öffnen des Sterilbehälters vereinfacht.

Diese Aufgabe wird durch einen modular aufgebauten Deckel für einen Sterilbehälter nach Anspruch 1, einen Sterilbehälter nach Anspruch 7 und eine Filterabdeckung nach Anspruch 8 gelöst. Vorteilhafte Ausführungsformen und Weiterbildungen sind in den Unteransprüchen beansprucht und/oder nachfolgend erläutert.

Die Erfindung betrifft zunächst einen modular aufgebauten Deckel für einen Sterilbehälter bzw. eines Sterilbehälters mit: einem Deckelbauteil, welches zumindest einen, insbesondere perforierten, Gasaustauschabschnitt aufweist, an welchem an einer Deckelinnenseite zumindest eine Filtereinrichtung anordbar/angeordnet ist, und zumindest einer, vorzugsweise (zumindest teilweise) transparenten, Filterabdeckung, welche den zumindest einen Gasaustauschabschnitt an einer Deckelaußenseite (vollständig) abdeckt bzw. mit welcher der zumindest eine Gasaustauschabschnitt (vollständig) abdeckbar ist, wobei das Deckelbauteil zumindest einen, insbesondere standardisierten, Clipaufnahmeabschnitt und die Filterabdeckung zumindest einen, insbesondere standardisierten, Clipabschnitt aufweisen (oder umgekehrt), über welche die Filterabdeckung und das Deckelbauteil zueinander formschlüssig, insbesondere durch Aufklipsen, befestigbar sowie werkzeuglos montierbar und demontierbar sind.

Ein zentraler Aspekt der vorliegenden Erfindung ist dabei der modulare Aufbau des Deckels. Erfindungsgemäß stellen das Deckelbauteil und die Filterabdeckung zwei separate Bauteile dar, welche einem Anwender sowohl in einem montierten Zustand als auch in einem demontierten/separaten Zustand zur Verfügung gestellt werden können. Das Deckelbauteil ist bevorzugt ein Bauteil, welches angepasst ist, ein wannenartiges Behälterteil eines Sterilbehälters vollständig abzudecken und zu verschließen. Die Filterabdeckung/Außenabdeckung dient einem Schutz der Filtereinrichtung, welche beispielsweise als Filter, Filterelement, Kombination aus Filterhalter und Filterelement etc. ausgebildet sein kann, vor mechanischen Einflüssen (z.B. spitze/scharfkantige Gegenstände, welche den Filter durchbohren oder einschneiden können) und verhindert/vermindert ein Eindringen von Flüssigkeiten in den Sterilbehälter. Die Filtereinrichtung ist bevorzugt an einer Deckelinnenseite/Sterilbehälterinnenseite an einem Gasaustauschabschnitt des Deckelbauteils anordbar und bedeckt diesen Gasaustauschabschnitt, welcher weiter bevorzugt eine Vielzahl von Perforationen/Löchern aufweist, vollständig, so dass ein Gasaustausch zwischen einer Sterilbehälter-/ Deckelinnenseite und einer Sterilbehälter-/ Deckelaußenseite (ausschließlich) über die Filtereinrichtung erfolgen kann. Der Gasaustauschabschnitt ist im montierten Zustand der Filterabdeckung an dem Deckelbauteil bevorzugt vollständig (über seine gesamte Fläche) von der Filterabdeckung abgedeckt.

Weiter bevorzugt weist das Deckelbauteil zumindest eine, vorzugsweise zwei, standardisierte Aufnahme(n)/ zumindest einen, vorzugsweise zwei, standardisierte(n) Clipaufnahmeabschnitt(e) auf, welche/welcher geeignet ist/sind, Filterabdeckungen mit unterschiedlichen Eigenschaften, welche jeweils zumindest einen, vorzugsweise zwei, standardisierte(n) Eingriffsabschnitt(e)/ zumindest einen, vorzugsweise zwei, standardisierte(n) Clipabschnitt(e) aufweisen, darin formschlüssig aufzunehmen. Beispielsweise können einem Anwender verschiedene Filterabdeckungen, welche sich in Material oder Kosten, in ihren mechanischen Eigenschaften, Dichtigkeitseigenschaften bzw. in ihrem bevorzugten Verwendungszweck (beispielsweise unterschiedliche Sterilisationsverfahren) unterscheiden, zur Verfügung gestellt werden. Die verschiedenen Filterabdeckungen sind erfindungsgemäß wahlweise austauschbar, so dass eine in ihren Eigenschaften an den jeweiligen Anwendungsbereich angepasste Filterabdeckung verwendet werden kann.

Dadurch, dass die Filterabdeckung und das Deckelbauteil zueinander formschlüssig, befestigbar sowie werkzeuglos/ ohne Verwendung von Werkzeugen montierbar und demontierbar sind, wird eine einfache Handhabbarkeit sowie eine beliebige Nachrüstbarkeit mit weiterentwickelten Filterabdeckungen erreicht. Weiterhin kann nach erfolgter Sterilisation die Filterabdeckung in einfacher Weise händisch von dem Deckelbauteil abgenommen werden und ein Zustand der Filtereinrichtung durch einen Anwender überprüft werden, ohne dass dieser den Sterilbehälter öffnen muss. Beispielsweise kann der Anwender sehen, ob die Filtereinrichtung eingelegt ist, möglicherweise beschädigt ist, oder, wenn an der Filtereinrichtung ein Prozessindikator aufgebracht ist, kontrollieren, ob die Filtereinrichtung einem sterilisationsähnlichen Prozess unterzogen wurde. Bevorzugt wird die Filterabdeckung daher nach jedem Sterilisationsvorgang demontiert und wieder montiert, was auch mit einer verbesserten Reinigung von sowohl Deckelbauteil als auch Filterabdeckung einhergeht.

Die Filterabdeckung des modular aufgebauten Deckels kann vorzugsweise einstückig (zumindest teilweise) aus einem transparenten Kunststoff, vorzugsweise im Spitzgussverfahren, hergestellt sein. Dies hat den weiteren Vorteil, dass ein Anwender die Filtereinrichtung auch im montierten Zustand der Filterabdeckung an dem Deckelbauteil einsehen kann. Weiterhin wird die Filterabdeckung dadurch kostengünstig sowie einfach handhabbar und herstellbar realisiert.

Grundsätzlich hat ein gattungsgemäßes Deckelbauteil eines Sterilbehälters (wie auch des vorliegenden, erfindungsgemäßen Sterilbehälters), wie vorstehend bereits beschrieben, eine im Wesentlichen flache/ebene Oberseite/Deckelaußenseite (nachfolgend auch als Deckelbauteilhauptebene bezeichnet), die von einem zumeist/optional vorzugsweise durch Bördeln/Tiefziehen daran (stoffeinstückig) ausgeformten Rahmen umgeben ist, der so dimensioniert sein kann, dass er bei Aufsetzen des Deckelbauteils auf das wannenartige Behälterteil dessen Rand außenseitig umgreift. Des Weiteren definiert der an der Deckelaußenseite sich ausbildende Gasaustauschabschnitt eine Ebene, welche zur Deckelbauteilhauptebene erhaben/höhenversetzt ist. Die Deckelbauteilhauptebene sowie die vom Gasaustauschabschnitt definierte/aufgespannte Ebene sind über einen rahmenförmig, den Gasaustauschabschnitt umgebenden Sockel/Übergangsabschnitt miteinander verbunden, der vorzugsweise stoffeinstückig an der Deckelbauteilhauptebene ausgebildet ist.

Gemäß der Erfindung ist an dem bevorzugt standardisierten Clipaufnahmeabschnitt des Deckelbauteils nunmehr eine Aussparung oder griffschalenartige Vertiefung/Mulde (bezüglich der Deckelbauteilhauptebene) vorgesehen, an welcher das Deckelbauteil, insbesondere über daran ausgeformte Aussparungs-/Muldenseitenflächen oder Flanken, von der Deckelbauteilhauptebene zu einer gegenüber der Deckelbauteilhauptebene um eine erste Höhe nach unten (hin zu einer Deckelinnenseite/Sterilbehälterinnenseite) versetzten, (ggf. parallel zur Deckelbauteilhauptebene verlaufenden) Aussparungsgrundfläche und über den, zumindest in diesem Bereich hinterschnittenen/zurückgenommenen bzw. S-förmigen Übergangsabschnitt von der Aussparungsgrundfläche zu dem zu der Deckelbauteilhauptebene (ggf. ebenso parallelen), insbesondere um eine zweite Höhe gegenüber der Deckelbauteilhauptebene nach oben (hin zu einer Deckelaußenseite/Sterilbehälteraußenseite) versetzten, Gasaustauschabschnitt übergeht.

Bevorzugt sind an sich gegenüberliegenden Seiten des Gasaustauschabschnitts jeweils eine Griffschale oder Mulde an der Deckelbauteilhauptebene ausgeformt, deren dem Gasaustauschabschnitt zugewandten Muldenrand jeweils einen Hinterschnitt ausbildet (S-förmiger Querschnitt), in den die bevorzugt ebenfalls sich gegenüberliegenden Clipabschnitte der Filterabdeckung (federelastisch) bei dessen Aufsetzen auf den Gasaustauschabschnitt bzw. den Übergangsabschnitt/Sockel verrastend eingreifen (etwa nach Art einer allgemein bekannten Frischhaltedose mit Deckel). Soll die Filterabdeckung wieder entfernt werden, muss lediglich im Bereich der Griffschalen/Mulden der untere (zur Deckelbauteilhauptebene zugewandte) Randbereich der Filterabdeckung mit den Fingerspitzen ergriffen und die Filterabdeckung elastisch über den jeweiligen Hinterschnitt hinweg abgezogen werden.

Bevorzugt ist der (standardisierte) Clipabschnitt der Filterabdeckung des modular aufgebauten Deckels als ein C-förmig/klammerartig verlaufender Seitenabschnitt/Endabschnitt/Leiste der Filterabdeckung ausgebildet und ist an dem C-förmig verlaufenden Seitenabschnitt ein seitlich nach außen vorstehender Greifabschnitt angeordnet, welcher bei einer Montage und einer Demontage der Filterabdeckung an dem Deckelbauteil infolge der in diesem Bereich ausgebildeten Griffschale/Mulde manuell betätigbar ist, wie dies vorstehend bereits beschrieben wurde.

Der Clipabschnitt der Filterabdeckung des modular aufgebauten Deckels ist bevorzugt an einem Seiten-/ End-/ Randabschnitt der Filterabdeckung als klammerartig/ C-förmig von einer Oberseite der Filterabdeckung (nachfolgend auch als Filterabdeckungshauptebene bezeichnet) weg gebogener Abschnitt ausgebildet. In anderen Worten ausgedrückt wird die Filterabdeckung an zumindest einer Seite/ einem Rand/ einem Ende von dem Clipabschnitt zumindest abschnittsweise seitlich begrenzt.

Der Greifabschnitt erstreckt sich vorzugsweise parallel zu der Filterabdeckungshauptebene nach außen von dem Clipabschnitt weg.

Es ist dabei vorteilhaft, wenn im montierten Zustand der Filterabdeckung an dem Deckelbauteil der klammerartig verlaufende Seitenabschnitt/Endabschnitt der Filterabdeckung, welcher den Clipabschnitt ausbildet, in den hinterschnittenen Übergangsabschnitt des Clipaufnahmeabschnitts des Deckelbauteils eingreift bzw. eingeklipst ist, wie dies ebenfalls vorstehend bereits angedeutet wurde.

Ein Aufklipsen/ Einklipsen der Filterabdeckung in/auf das Deckelbauteil stellt, insbesondere wenn der Clipabschnitt und der Greifabschnitt der Filterabdeckung an einer Seite/ einem Rand/ einem Ende der Filterabdeckung angordnet/vorgesehen sind, eine geeignete mechanische Verbindung bereit, mit welcher die Filterabdeckung und das Deckelbauteil zueinander/aneinander formschlüssig befestigt sowie werkzeuglos montiert und demontiert werden können.

In anderen Worten ist der Clipabschnitt bevorzugt als ein klammerartig verlaufender Seitenabschnitt/Endabschnitt der Filterabdeckung ausgebildet und weist der Clipaufnahmeabschnitt einen S-förmigen (hinterschnittenen) Abschnitt auf, wobei im montierten Zustand der Filterabdeckung an dem Deckelbauteil der klammerartig verlaufende Seitenabschnitt der Filterabdeckung in den hinterschnittenen Abschnitt des Clipaufnahmeabschnitts eingreift/ eingeklipst ist.

Ein vorteilhaftes Ausführungsbeispiel sieht vor, dass die Filterabdeckung des modular aufgebauten Deckels zwei Clipabschnitte aufweist, welche an gegenüberliegenden, vorzugsweise parallelen, Seiten und/oder Enden der Filterabdeckung angeordnet sind, und das Deckelbauteil zwei Clipaufnahmeabschnitte aufweist, welche an gegenüberliegenden, vorzugsweise parallelen, Seiten und/oder Enden des Gasaustauschabschnitts angeordnet sind, und im montierten Zustand der Filterabdeckung an dem Deckelbauteil die zwei Clipabschnitte in die zwei Clipaufnahmeabschnitte eingreifen/ eingeklipst sind.

Bei der Montage der Filterabdeckung an dem Deckelbauteil wird bevorzugt zunächst der erste Clipabschnitt in den ersten Clipaufnahmeabschnitt eingesetzt. Anschließend wird der zweite Clipabschnitt in den zweiten Clipaufnahmeabschnitt durch händische Betätigung des Greifabschnitts unter reversibler elastischer Aufbiegung der Filterabdeckung gedrückt. Bei der Demontage der Filterabdeckung von dem Deckelbauteil wird die Filterabdeckung an einem Mittelabschnitt derselben nach unten gedrückt und wird an einem der zwei Greifabschnitte gezogen, wodurch eine reversible elastische Aufbiegung der Filterabdeckung und eine Abnahme derselben von dem Deckelbauteil bewirkt wird.

Besonders bevorzugt ist die Filterabdeckung aus einem amorphen Thermoplast hergestellt sowie transparent und ermöglicht ein visuelles Überprüfen der Filtereinrichtung von der Deckelaußenseite ohne vorheriges Demontieren der Filterabdeckung von dem Deckelbauteil.

Ist die Filterabdeckung transparent, kann ein Zustand derselben durch einen Anwender überprüft werden, ohne dass die Filterabdeckung von dem Deckelbauteil demontiert werden muss. Beispielsweise kann der Anwender sehen, ob die Filtereinrichtung eingelegt ist, möglicherweise beschädigt ist, oder, wenn an der Filtereinrichtung ein Prozessindikator aufgebracht ist, kontrollieren, ob die Filtereinrichtung einem sterilisationsähnlichem Prozess unterzogen wurde. Ist die Filterabdeckung ferner aus einem amorphen Thermoplast hergestellt, wird zum einen die zur Montage und Demontage benötigte Elastizität der Filterabdeckung bei ausreichender Steifigkeit und Festigkeit (für eine Befestigung der Filterabdeckung an dem Deckelbauteil) bereitgestellt, zum anderen wird eine kostengünstige, einstückige Herstellung der gesamten Filterabdeckung im Spritzgussverfahren ermöglicht.

Die Erfindung betrifft weiterhin einen Sterilbehälter mit einem wannenartigen Behälterteil und einem Deckel wie voranstehend beschrieben.

Außerdem betrifft die Erfindung eine Filterabdeckung für einen Deckel wie voranstehend beschrieben, zum vollständigen Abdecken von zumindest einem an einem Deckelbauteil des Sterilbehälterdeckels vorgesehenen, insbesondere perforierten, Gasaustauschabschnitt an einer Deckelaußenseite, welche einstückig aus, vorzugsweise transparentem, Kunststoff hergestellt ist. Die Filterabdeckung weist zumindest zwei an gegenüberliegenden Seiten und/oder Enden der Filterabdeckung angeordnete, insbesondere standardisierte, Clipabschnitte auf, welche dazu angepasst sind, formschlüssig, insbesondere durch Aufklipsen, in zwei an dem Deckelbauteil an gegenüberliegenden Seiten und/oder Enden des Gasaustauschabschnitts angeordneten Clipaufnahmeabschnitten einzugreifen, und über welche die Filterabdeckung werkzeuglos an dem Deckelbauteil montierbar und demontierbar ist. Die Filterabdeckung ist eine rechteckige Platte, deren Höhe im Verhältnis zu ihrer Länge und Breite klein ist, und die Clipabschnitte sind als klammerartig bzw. C-förmig von einer Filterabdeckungshauptebene weg gebogene Abschnitte ausgebildet.

Bevorzugt ist an dem C-förmig/ klammerartig verlaufenden Seitenabschnitt/ Endabschnitt der Filterabdeckung ein seitlich nach außen vorstehender Greifabschnitt angeordnet, welcher bei der Montage und Demontage der Filterabdeckung an dem Deckelbauteil manuell betätigbar ist.

Die Filterabdeckung weist zwei Clipabschnitte auf, welche an gegenüberliegenden, vorzugsweise parallelen, Seiten und/oder Enden der Filterabdeckung angeordnet sind. Die zwei Clipabschnitte sind angepasst, im montierten Zustand der Filterabdeckung an dem Deckelbauteil in zwei an dem Deckelbauteil an gegenüberliegenden, vorzugsweise parallelen, Seiten und/oder Enden des Gasaustauschabschnitts angeordneten Clipaufnahmeabschnitten formschlüssig einzugreifen.

In vorteilhafter Weise ist Filterabdeckung ferner aus einem amorphen Thermoplast hergestellt sowie transparent und ermöglicht ein visuelles Überprüfen einer an einer Deckelinnenseite des Gasaustauschabschnitts anordbaren Filtereinrichtung von einer Deckelaußenseite ohne vorheriges Demontieren der Filterabdeckung von dem Deckelbauteil.

### Kurzbeschreibung der Figuren

Die Erfindung wird nachfolgend mit Hilfe von Figuren weiter erläutert. Es zeigen:
- Fig. 1: eine perspektivische Ansicht eines Sterilbehälters mit Deckel und Aufnahmebehälter;
- Fig. 2: eine vergrößerte perspektivische Ansicht einer an einem Deckelbauteil angebrachten Filterabdeckung; und
- Fig. 3: eine an der Achse A-A in Fig. 2 geschnittene perspektivische Ansicht der an dem Deckelbauteil angebrachten Filterabdeckung.

### Figurenbeschreibung

Die Figuren sind lediglich schematischer Natur und dienen ausschließlich dem Verständnis der Erfindung. Gleiche Elemente sind dabei mit denselben Bezugszeichen versehen.

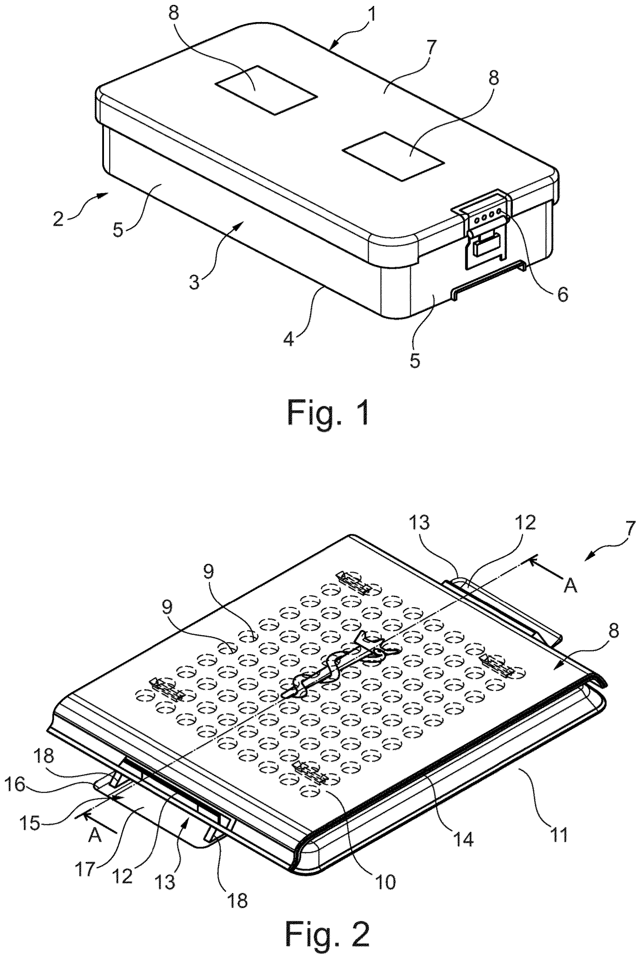

In Fig. 1 ist eine perspektivische Ansicht eines erfindungsgemäßen Deckels 1 für einen Sterilbehälter 2 dargestellt, welcher einen wannenartigen Aufnahmebehälter 3 verschließt. Der Aufnahmebehälter 3 besteht dabei aus einem Behälterboden 4 und von diesem aufsteigende Behälterwände 5. Über einen Verschluss 6 werden der Aufnahmebehälter 3 und der Deckel 1 verschlossen. Der Deckel 1 weist ein Deckelbauteil 7 auf, welches außen von zwei Filterabdeckungen 8 bedeckt ist. Erfindungsgemäß können auch eine Filterabdeckung 8 (ein Gasaustauschabschnitt an dem Deckelbauteil 7) oder mehr als zwei Filterabdeckungen 8 (mehr als zwei Gasaustauschabschnitte an dem Deckelbauteil 7) vorgesehen sein. Unter den Filterabdeckungen 8 sind an dem Deckelbauteil 7 in Fig. 1 nicht dargestellte perforierte bzw. lochplattenartige Abschnitte vorgesehen. Das Deckelbauteil 7 bedeckt den Aufnahmebehälter 3 vollständig. Das Deckelbauteil 7 und die Filterabdeckung 8 sind separate Bauteile.

Fig. 2 zeigt eine vergrößerte perspektivische Ansicht von einer an dem Deckelbauteil 7 angebrachten Filterabdeckung 8. Die in Fig. 2 dargestellte Filterabdeckung 8 ist transparent bzw. durchsichtig und beispielsweise aus einem amorphen Kunststoff Thermoplast im Spritzgussverfahren hergestellt. Aufgrund der Transparenz der Filterabdeckung 8 sind Perforationen/ Löcher 9 sichtbar, welche an einem Gasaustauschabschnitt 10 des Deckelbauteils 7 vorgesehen sind. Der Perforationen/ Löcher 9 des Gasaustauschabschnitts 10 erlauben einen Medienaustausch während einer Sterilisation. In Fig. 2 ist bereits zu sehen, dass der Gasaustauschabschnitt 10 ein gegenüber einer Deckelbauteilhauptebene 11 vorragender Abschnitt ist. Die Filterabdeckung 8 ist rechteckig und platten- bzw. scheibenartig (als eine dünne Platte/Scheibe) ausgebildet. Insbesondere ist eine Höhe/Dicke der Filterabdeckung 10 im Verhältnis zu ihrer Länge und Breite vernachlässigbar klein.

An zwei einander gegenüberliegenden, vorzugsweise kürzeren, (Rechteck-)Seiten weist die Filterabdeckung 8 jeweils einen Clipabschnitt 12 auf, welcher an einem Mittelabschnitt der jeweiligen (Rechteck-)Seite vorgesehen ist. Die Clipabschnitte 12 der Filterabdeckung 8 sind jeweils in Clipaufnahmeabschnitte 13 des Deckelbauteils 7 formschlüssig aufgenommen. An den (Rechteck-)Seiten der Filterabdeckung 8, welche die Clipabschnitte 12 aufweisen, ist die Filterabdeckung (um)gebogen und abschnittsweise in Anlage mit dem vorspringenden Gasaustauschabschnitt 10 des Deckelbauteils sowie endseitig mit der Deckelbauteilhauptebene 11. An den beiden anderen, einander ebenso gegenüberliegenden, vorzugsweise längeren, (Rechteck-) Seiten ist die Filterabdeckung 8 von dem Gasaustauschabschnitt 10, insbesondere um etwa 2 mm, beabstandet angeordnet. Während einer Sterilisation kann dann ein Medienaustausch insbesondere über den zwischen der Filterabdeckung 8 und dem Gasaustauschabschnitt 10 vorgesehenen schmalen Spalt 14 erfolgen.

Jeder Clipaufnahmeabschnitt 13 weist unter anderem eine Aussparung 15 auf, welche sich aus Aussparungsseitenflächen 16 und einer Aussparungsgrundfläche 17 zusammensetzt. An zwei einander gegenüberliegenden Aussparungsseitenflächen 16 sind Vorsprünge 18 der Filterabdeckung 8 geführt. In anderen Worten sind die Vorsprünge 18 der Filterabdeckung 8 im montierten Zustand der Filterabdeckung 8 an dem Deckelbauteil 7 angrenzend an die zwei einander gegenüberliegenden Aussparungsseitenflächen 16 angeordnet.

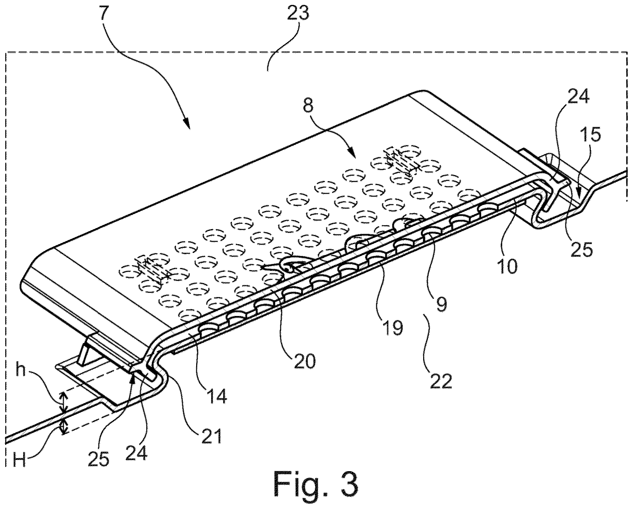

Fig. 3 zeigt eine an der Achse A-A in Fig. 2 geschnittene perspektivische Ansicht der an dem Deckelbauteil 7 angebrachten Filterabdeckung 8. Es ist hier zunächst erkennbar, dass an einer Unterseite/ zu einer Deckelinnenseite/Sterilbehälterinnenseite hin des Gasaustauschabschnitts 10, welcher die Löcher/Perforationen 9 aufweist, ein Filter 19 angeordnet ist, welcher als rechteckiges, dünnes Filterelement ausgebildet ist und alle Löcher/Perforationen 9 des Gasaustauschabschnitts vollständig bedeckt. Weiterhin wird in Fig. 3 deutlicher, dass die Filterabdeckung 8 und der Gasaustauschabschnitt 10 voneinander beabstandet sind bzw. in anderen Worten zwischen dem Gasaustauschabschnitt 10 und einer Filterabdeckungshauptebene 20 ein Spalt 14 ausgebildet ist.

In Fig. 3 wird weiterhin deutlich, dass die Aussparung 15 neben den Aussparungsseitenflächen 16 und der Aussparungsgrundfläche 17 einen S-förmigen Übergangsabschnitt 21 aufweist. In anderen Worten geht das Deckelbauteil 7 an der Aussparung 15 über Aussparungsseitenflächen 16 von der Deckelbauteilhauptebene 11 zu der Aussparungsgrundfläche 17 und über den S-förmigen Übergangsabschnitt 21 von der Aussparungsgrundfläche 17 zu dem Gasaustauschabschnitt 10 über. Die Aussparungsgrundfläche 17 ist gegenüber der Deckelbauteilhauptebene 11 um eine Höhe H nach unten/ hin zu einer Deckelinnenseite 22 versetzt. Der Gasaustauschabschnitt 10 ist gegenüber der Deckelbauteilhauptebene 11 um eine Höhe h nach oben/ hin zu einer Deckelaußenseite 23 versetzt. Aussparungsgrundfläche 17, Deckelbauteilhauptebene 11 und Gasaustauschabschnitt 10 sind zueinander parallel. Der S-förmige Übergangsabschnitt 21 verläuft S-förmig zwischen der Aussparungsgrundfläche 17 und dem Gasaustauschabschnitt 10.

Weiterhin geht aus Fig. 3 hervor, wie die Clipabschnitte 12 der Filterabdeckung 8 ausgebildet sind. Die Clipabschnitte 12 sind klammerförmig/ C-förmig verlaufende Endabschnitte/ (Rechteck-)Seitenabschnitte 24, welche sich an zwei gegenüberliegenden (Rechteck-)Seiten der Filterabdeckung klammerartig/ C-förmig von der Filterabdeckungshauptebene 20 nach unten/ zu einer Deckelinnenseite 22 hin erstrecken. Von den klammerförmig/ C-förmig verlaufenden (Rechteck-) Seitenabschnitten 24 erstreckt sich ein Greifabschnitt 25 seitlich nach außen vorspringend weg. Der Greifabschnitt 25 ist parallel zur Filterabdeckungshauptebene 20.

Im montierten Zustand der Filterabdeckung 8 an dem Deckelbauteil 7 sind die klammerförmig/ C-förmig verlaufenden (Rechteck-)Seitenabschnitte 24 der Filterabdeckung 8 auf die S-förmigen Übergangsabschnitte 21 des Deckelbauteils 7 eingeklipst/ aufgeklipst bzw. klammern sich die C-förmig verlaufenden (Rechteck-) Seitenabschnitte 24 an die S-förmigen Übergangsabschnitte 21, so dass die Filterabdeckung 8 und das Deckelbauteil 7 dadurch formschlüssig miteinander verbunden werden. Das Aufklipsen/ Einklipsen der Clipabschnitte 12 der Filterabdeckung 8 in die Clipaufnahmeabschnitte 13 des Deckelbauteils 7 wird insbesondere durch die Elastizität des verwendeten amorphen Thermoplasten ermöglicht.

### Bezugszeichenliste

- 1: Deckel
- 2: Sterilbehälter
- 3: wannenartiger Aufnahmebehälter
- 4: Behälterboden
- 5: Behälterwand
- 6: Verschluss
- 7: Deckelbauteil
- 8: Filterabdeckung
- 9: Perforationen/Löcher
- 10: Gasaustauschabschnitt
- 11: Deckelbauteilhauptebene
- 12: Clipabschnitt
- 13: Clipaufnahmeabschnitt
- 14: Spalt
- 15: Aussparung
- 16: Aussparungsseitenfläche
- 17: Aussparungsgrundfläche
- 18: Vorsprung
- 19: Filter
- 20: Filterabdeckungshauptebene
- 21: S-förmiger Übergangsabschnitt
- 22: Deckelinnenseite
- 23: Deckelaußenseite
- 24: C-förmig verlaufender Seitenabschnitt
- 25: Greifabschnitt

## Patentansprüche

1. Modular aufgebauter Deckel (1) für einen Sterilbehälter (2) mit:
einem Deckelbauteil (7), welches zumindest einen Gasaustauschabschnitt (10) aufweist, an welchem zumindest eine Filtereinrichtung (19) anordbar ist, und
zumindest einer Filterabdeckung (8), mit welcher der zumindest eine Gasaustauschabschnitt (10) an einer Deckelaußenseite (23) abdeckbar ist, wobei das Deckelbauteil (7) zumindest einen Clipaufnahmeabschnitt (13) und die Filterabdeckung (8) zumindest einen Clipabschnitt (12) aufweisen, über welche die Filterabdeckung (8) an das Deckelbauteil (7) formschlüssig, insbesondere durch Aufklipsen, befestigbar sowie werkzeuglos montierbar und demontierbar ist, **dadurch gekennzeichnet, dass**
an dem Clipaufnahmeabschnitt (13) des Deckelbauteils (7) eine muldenförmige Aussparung (15) vorgesehen ist, an welcher das Deckelbauteil (7) von einer Deckelbauteilhauptebene (11) zu einer gegenüber der Deckelbauteilhauptebene (11) zu einer Deckelinnenseite (22) hin versetzten Aussparungsgrundfläche (17) und von der Aussparungsgrundfläche (17) über einen S-förmigen Übergangsabschnitt (21) zu dem gegenüber der Deckelbauteilhauptebene (11) zu einer Deckelaußenseite (23) hin versetzten Gasaustauschabschnitt (10) übergeht.

2. Modular aufgebauter Deckel (1) nach Anspruch 1, wobei der Clipabschnitt (12) der Filterabdeckung (8) als ein klammerartig verlaufender Seitenabschnitt (24) der Filterabdeckung (8) ausgebildet ist und an dem klammerartig verlaufenden Seitenabschnitt (24) ein seitlich nach außen vorstehender Greifabschnitt (25) angeordnet ist, welcher bei der Montage und Demontage der Filterabdeckung (8) an dem Deckelbauteil (7) manuell betätigbar ist.

3. Modular aufgebauter Deckel (1) nach Anspruch 1 und 2, wobei im montierten Zustand der Filterabdeckung (8) an dem Deckelbauteil (7) der klammerartig verlaufende Seitenabschnitt (24) der Filterabdeckung (8) in den S-förmigen Übergangsabschnitt (21) des Deckelbauteils (7) formschlüssig eingreift.

4. Modular aufgebauter Deckel (1) nach Anspruch 1, wobei der Clipabschnitt (12) als ein klammerartig verlaufender Seitenabschnitt (24) der Filterabdeckung (8) ausgebildet ist und der Clipaufnahmeabschnitt (13) den S-förmigen Übergangsabschnitt (21) aufweist, wobei im montierten Zustand der Filterabdeckung (8) an dem Deckelbauteil (7) der klammerartig verlaufende Seitenabschnitt (24) der Filterabdeckung (8) in den S-förmigen Übergangsabschnitt (21) des Clipaufnahmeabschnitts (13) eingreift.

5. Modular aufgebauter Deckel (1) nach einem der vorhergehenden Ansprüche, wobei die Filterabdeckung (8) zwei Clipabschnitte (12) aufweist, welche an gegenüberliegenden, vorzugsweise parallelen, Seiten und/oder Enden der Filterabdeckung (8) angeordnet sind, und das Deckelbauteil (7) zwei Clipaufnahmeabschnitte (13) aufweist, welche an gegenüberliegenden, vorzugsweise parallelen, Seiten und/oder Enden des Gasaustauschabschnitts (10) angeordnet sind, und im montierten Zustand der Filterabdeckung (8) an dem Deckelbauteil (7) die zwei Clipabschnitte (12) in die zwei Clipaufnahmeabschnitte (13) eingreifen.

6. Modular aufgebauter Deckel (1) nach einem der vorhergehenden Ansprüche, wobei die Filterabdeckung (8) aus einem amorphen Thermoplast hergestellt sowie transparent ist zum visuellen Überprüfen der Filtereinrichtung (19) von der Deckelaußenseite (23) ohne vorheriges Demontieren der Filterabdeckung (8) von dem Deckelbauteil (7).

7. Sterilbehälter (2) mit einem wannenartigen Behälterteil (3) und einem Deckel (1) nach einem der vorhergehenden Ansprüche.

8. Filterabdeckung (8) für einen Deckel (1) nach einem der Ansprüche 1 bis 6, zum vollständigen Abdecken von zumindest einem an einem Deckelbauteil (7) des Sterilbehälterdeckels (1) vorgesehenen Gasaustauschabschnitt (10) an einer Deckelaußenseite (23), welche einstückig aus Kunststoff hergestellt ist, **dadurch gekennzeichnet, dass** die Filterabdeckung (8) zumindest zwei an gegenüberliegenden Seiten und/oder Enden der Filterabdeckung (8) angeordnete Clipabschnitte (12) aufweist, welche dazu angepasst sind, formschlüssig, insbesondere durch Aufklipsen, in zwei an dem Deckelbauteil (7) an gegenüberliegenden Seiten und/oder Enden des Gasaustauschabschnitts (10) angeordneten Clipaufnahmeabschnitten (13) einzugreifen, und über welche die Filterabdeckung (8) werkzeuglos an dem Deckelbauteil (7) montierbar und demontierbar ist, wobei
die Filterabdeckung (8) eine rechteckige Platte ist, deren Höhe im Verhältnis zu ihrer Länge und Breite klein ist, und die Clipabschnitte (12) als klammerartig bzw. C-förmig von einer Filterabdeckungshauptebene weg gebogene Abschnitte ausgebildet sind.

9. Filterabdeckung (8) nach Anspruch 8, wobei die Clipabschnitte als klammerartig verlaufende Seitenabschnitte (24) der Filterabdeckung (8) ausgebildet sind und an den klammerartig verlaufenden Seitenabschnitten (24) jeweils ein seitlich nach außen vorstehender Greifabschnitt (25) angeordnet ist, welcher bei der Montage und Demontage der Filterabdeckung (8) an dem Deckelbauteil (7) manuell betätigbar ist.

10. Filterabdeckung (8) nach Anspruch 8 oder 9, welcher ferner aus einem amorphen Thermoplast hergestellt sowie transparent ist, zum visuellen Überprüfen einer an einer Deckelinnenseite (22) des Gasaustauschabschnitts (10) anordbaren Filtereinrichtung (19) von einer Deckelaußenseite (23) ohne vorheriges Demontieren der Filterabdeckung (8) von dem Deckelbauteil (7).

## Claims

1. A modularly structured lid (1) for a sterile container (2) comprising:
a lid component (7) which has at least one gas exchange portion (10) on which at least one filter device (19) can be arranged, and
at least one filter covering (8) by which the at least one gas exchange portion (10) can be covered on an outside of the lid (23), wherein
the lid component (7) has at least one clip receiving portion (13) and the filter covering (8) has at least one clip portion (12) by which the filter covering (8) can be positively fastened to the lid component (7), in particular through clipping on, and can be mounted and dismounted without tools, **characterized in that**
at the clip receiving portion (13) of the lid component (7), a trough-shaped recess (15) is provided at which the lid component (7) merges from a lid component main plane (11) into a recess base area (17) offset from the lid component main plane (11) towards an inside of the lid (22), and merges from the recess base area (17) via an S-shaped transition portion (21) into the gas exchange portion (10) offset from the lid component main plane (11) towards an outside of the lid (23).

2. The modularly structured lid (1) according to claim 1, wherein the clip portion (12) of the filter covering (8) is configured as a clamp-shaped side portion (24) of the filter covering (8), and on the clamp-shaped side portion (24) there is arranged a laterally outwardly projecting grip portion (25) which is manually operable when the filter covering (8) is mounted and dismounted on the lid component (7).

3. The modularly structured lid (1) according to claim 1 or 2, wherein when the filter covering (8) is mounted on the lid component (7), the clamp-shaped side portion (24) of the filter covering (8) positively engages in the S-shaped transition portion (21) of the lid component (7).

4. The modularly structured lid (1) according to claim 1, wherein the clip portion (12) is a clamp-shaped side portion (24) of the filter covering (8) and the clip receiving portion (13) includes an S-shaped transition portion (21), wherein when the filter covering (8) is mounted on the lid component (7), the clamp-shaped side portion (24) of the filter covering (8) engages in the S-shaped transition portion (21) of the clip receiving portion (13).

5. The modularly structured lid (1) according to any of the preceding claims,
wherein the filter covering (8) has two clip portions (12) which are arranged on opposite, preferably parallel, sides and/or ends of the filter covering (8) and the lid component (7) has two clip receiving portions (13) which are arranged on opposite, preferably parallel, sides and/or ends of the gas exchange portion (10), and, when the filter covering (8) is mounted on the lid component (7), the two clip portions (12) engage in the two clip receiving portions (13).

6. The modularly structured lid (1) according to any of the preceding claims,
wherein the filter covering (8) is made from amorphous thermoplastic and is transparent for visually inspecting the filter device (19) from the outside of the lid (23) without previous dismounting of the filter covering (8) from the lid component (7).

7. A sterile container (2) comprising a trough-shaped container part (3) and a lid (1) according to any of the preceding claims.

8. A filter covering (8) for a lid (1) according to any of claims 1 to 6, for completely covering at least one gas exchange portion (10), which is provided on a lid component (7) of the sterile container lid (1) on an outside of the lid (23), which is made from plastics in one part, **characterized in that** the filter covering (8) has at least two clip portions (12) located on opposite sides and/or ends of the filter covering (8), which are adapted to positively engage, in particular through clipping on, in two clip receiving portions (13) which are arranged on the lid component (7) on opposite sides and/or ends of the gas exchange portion (10), and by which the filter covering (8) can be mounted and dismounted on the lid component (7) without tools,
the filter covering (8) comprising a rectangular plate, the height of which being small in relation to its length and width, and the clip portions (12) being formed as portions in the shape of a clamp or C-shaped bent away from a filter covering main plane.

9. The filter covering (8) according to claim 8, wherein the clip portions are in the form of clamp-shaped side portions (24) of the filter covering (8), and on the clamp-shaped side portions (24) a laterally outwardly projecting grip portion (25) is arranged which can be manually operated when the filter covering (8) is mounted and dismounted on the lid component (7).

10. The filter covering (8) according to claim 8 or 9, which is further made from amorphous thermoplastic and is transparent, for visually inspecting a filter device (19), which can be arranged on an inside of the lid (22) of the gas exchange portion (10) from an outside of the lid (23) without previous dismounting of the filter covering (8) from the lid component (7).

## Revendications

1. Couvercle de conception modulaire (1) pour un récipient stérile (2) avec :
un composant de couvercle (7) qui présente au moins une section d'échange de gaz (10) sur laquelle au moins un appareil de filtre (19) peut être disposé, et
au moins un cache de filtre (8) avec lequel l'au moins une section d'échange de gaz (10) peut être couverte sur un côté extérieur de couvercle (23), dans lequel le composant de couvercle (7) présente au moins une section de logement de clip (13) et le cache de filtre (8) au moins une section de clip (12), par l'intermédiaire desquelles le cache de filtre (8) peut être fixé au composant de couvercle (7) par complémentarité de formes, en particulier par clipsage, et peut être montée et démontée sans outils, **caractérisé en ce que**
un évidement (15) en forme d'auge est prévu sur la section de logement de clip (13) du composant de couvercle (7), évidement sur lequel le composant de couvercle (7) passe d'un plan principal de composant de couvercle (11) à une surface de base d'évidement (17) décalée par rapport au plan principal de composant de couvercle (11) vers un côté intérieur de couvercle (22) et de la surface de base d'évidement (17) à la section d'échange de gaz (10) décalée par rapport au plan principal de composant de couvercle (11) vers un côté extérieur de couvercle (23) à travers une section de transition en forme de S (21).

2. Couvercle de conception modulaire (1) selon la revendication 1, dans lequel la section de clip (12) du cache de filtre (8) est conçue en tant que section latérale (24) du cache de filtre (8) s'étendant à la manière d'une pince, et une section de préhension (25) faisant saillie latéralement vers l'extérieur est disposée sur la section latérale (24) s'étendant à la manière d'une pince, section de préhension qui peut être actionnée manuellement lors du montage et du démontage du cache de filtre (8) sur le composant de couvercle (7).

3. Couvercle de conception modulaire (1) selon les revendications 1 et 2, dans lequel, à l'état monté du cache de filtre (8) sur le composant de couvercle (7), la section latérale (24) de la couverture de filtre (8) s'étendant à la manière d'une pince s'engage par complémentarité de formes dans la section de transition en forme de S (21) du composant de couvercle (7).

4. Couvercle de conception modulaire (1) selon la revendication 1, dans lequel la section de clip (12) est conçue en tant que section latérale (24) de la couverture de filtre (8) s'étendant à la manière d'une pince et la section de logement de clip (13) présente la section de transition en forme de S (21), dans lequel, à l'état monté du cache de filtre (8) sur le composant de couvercle (7), la section latérale (24) du cache de filtre (8) s'étendant à la manière d'une pince s'engage dans la section de transition en forme de S (21) de la section de logement de clip (13).

5. Couvercle de conception modulaire (1) selon l'une quelconque des revendications précédentes, dans lequel le cache de filtre (8) présente deux sections de clip (12) qui sont disposées sur des côtés et/ou des extrémités opposés, de préférence parallèles, du cache de filtre (8), et le composant de couvercle (7) présente deux sections de logement de clip (13) qui sont disposées sur des côtés et/ou des extrémités opposés, de préférence parallèles, de la section d'échange de gaz (10), et à l'état monté du cache de filtre(8) sur le composant de couvercle (7), les deux sections de clip (12) s'engagent dans les deux sections de logement de clip (13).

6. Couvercle de conception modulaire (1) selon l'une quelconque des revendications précédentes, dans lequel le cache de filtre (8) est fabriqué en thermoplastique amorphe et est transparent pour le contrôle visuel de l'appareil de filtre (19) à partir du côté extérieur de couvercle (23) sans démontage préalable du cache de filtre (8) du composant de couvercle (7).

7. Récipient stérile (2) avec une partie de récipient en forme d'auge (3) et un couvercle (1) selon l'une quelconque des revendications précédentes.

8. Cache de filtre (8) pour un couvercle (1) selon l'une quelconque des revendications 1 à 6, pour couvrir complètement au moins une section d'échange de gaz (10) prévue sur un composant de couvercle (7) du couvercle de récipient stérile (1) sur un côté extérieur de couvercle (23) qui est fabriqué d'un seul tenant en matière plastique, **caractérisé en ce que** le cache de filtre (8) présente au moins deux sections de clip (12) disposées sur des côtés et/ou extrémités opposés du cache de filtre (8), sections qui sont adaptées pour s'engager par complémentarité de formes, en particulier par clipsage, dans deux sections de logement de clip (13) disposées sur le composant de couvercle (7) sur des côtés et/ou des extrémités opposés de la section d'échange de gaz (10), et par l'intermédiaire desquelles le cache de filtre (8) peut être monté et démonté sans outils sur le composant de couvercle (7), dans lequel
le cache de filtre (8) est une plaque rectangulaire dont la hauteur est petite par rapport à sa longueur et à sa largeur, et les sections de clip (12) sont conçues en tant que sections pliées à la manière d'une pince ou en forme de C à l'écart d'un plan principal de la couverture de filtre.

9. Cache de filtre (8) selon la revendication 8, dans lequel les sections de clip sont conçues en tant que sections latérales (24) du cache de filtre (8) s'étendant à la manière d'une pince et respectivement une section de préhension (25) faisant saillie latéralement vers l'extérieur est disposée sur les sections latérales (24) s'étendant à la manière d'une pince, section qui peut être actionnée manuellement lors du montage et du démontage du cache de filtre (8) sur le composant de couvercle (7).

10. Cache de filtre (8) selon la revendication 8 ou 9 qui est en outre fabriqué à partir d'un thermoplastique amorphe et est transparent pour le contrôle visuel d'un appareil de filtre (19) pouvant être disposé sur un côté intérieur de couvercle (22) de la section d'échange de gaz (10) à partir d'un côté extérieur de couvercle (23) sans démontage préalable du cache de filtre (8) du composant de couvercle (7).
